# EUROPEAN PATENT APPLICATION

(11) **EP 4 534 551 A1**
(43) Date of publication of application: **09.04.2025**
(21) Application number: 23729797.3
(22) Date of filing: 23.05.2023
(51) Int. Cl.: C07K 14/395, C12P 21/02, C12N 15/62, C12N 15/81, C40B 40/02, C12N 9/02, C12N 9/12

(54) **YEAST AND PLASMID FOR SURFACE PROTEIN EXPOSURE**

(30) Priority: 24.05.2022 ES 202230441
(71) Applicant: Universitat Politècnica de València, 46022 Valencia (ES)
(72) Inventor: PASCUAL-AHUIR GINER, María Desamparados, 46022 Valencia (ES)
(74) Representative: Pons
(86) International application number: PCT/ES2023/070333
(87) International publication number: WO 2023/227815

(57) **Abstract**

The present invention relates to a recombinant yeast characterized in that its genome comprises a metabolite-inducible promoter in place of the native promoter of the AGA1 gene, to a plasmid comprising (i) a metabolite-inducible promoter operatively linked the nucleotide sequence coding for a fusion protein comprising a protein of interest, and to the combined use of said yeast and plasmid in a Yeast Surface Display method.

## Description

The present invention refers to a recombinant yeast, a plasmid, and the use of both in the Yeast Surface Display (YSD) methodology. Therefore, the present invention is framed in the biotechnology sector, specifically in genetic engineering and in the elaboration of protein libraries of interest.

### BACKGROUND OF THE INVENTION

The yeast surface display (YSD) technique has become a versatile tool for industrial and research applications. In 1997, K. Dane Wittrup and colleagues described the first YSD library construction and selection processes (Boder ET, Wittrup KD. 1997. Nat Biotechnol. 15(6): 553-7), and since then, the field has experienced explosive growth. It is now widely used for antibody screening, protein engineering, studying protein-ligand interactions, and even in biocatalysis (Cherf GM, Cochran JR, 2015. Methods Mol Biol; 1319:155-75). Wittrup's system is based on the controlled expression of a cell wall-localized protein fused to a specific protein or library. The result is the coating of the yeast cell wall with the desired protein so that each yeast contains the genetic material that produces it. From Wittrup's original design, many plasmids with different properties have been developed, which in most cases have complicated their use and restricted their use to specific experiments (Yi L. et al. 2013. Proc Natl Acad Sci USA. 110(8): 7229-34).

The most commonly used system involves the cell wall agglutinins AGA1 and AGA2, as described, for example, in application WO2010069913 A1. Application US201010184136 A1 describes a method for improving the secretion efficiency of a heterologous protein in a yeast expression system. The method comprises (i) transforming a yeast defective in the gene encoding Gal2 with a recombinant gene construct comprising a galactose-inducible promoter, a secretion signal sequence, and a gene encoding a heterologous protein and (ii) culturing the transformed yeast under conditions that allow control of the galactose-inducible promoter. Yang, X. et al., 2019 (Microb Cell Fact 18:85. https://doi.org/10.1186/s12934-019-1133-x) describe a platform for heterologous protein expression on the surface of S. cerevisiae with higher efficiency than the classical Aga1p-Aga2p agglutinin system. The efficacy of this platform is based on employing a single surface-anchoring protein (Agalp) instead of two, thus avoiding the fragility of disulfide bridges to redox conditions.

However, these systems should achieve optimal cell surface expression of the protein of interest in sufficient quantity to be applied in an industrial context. Higher exposure is essential to obtain optimal results, and these must be controlled to conduct better reproducibility. Therefore, there is a need for the state of art to optimize, simplify and make a system more flexible for protein exposure on the cell surface, and offer a controlled, scalable, and automation-enabled system for industrial application.

### DESCRIPTION OF THE INVENTION

The inventors have observed that the expression of recombinant proteins on the yeast surface is increased when a metabolite-inducible promoter, such as the galactose-inducible promoter, replaces the native promoter that regulates AGA1 expression. The expression of recombinant proteins on the surface of yeast is increased compared to those yeasts that present the native promoter (see Examples). Thus, the inventors have developed a yeast that, when used in the Yeast Surface Display (YSD) technique, allows the optimization of the surface exposure of the protein of interest, thus overcoming the drawbacks of other existing methods in the state of the art.

In addition, the inventors have also developed a set of plasmids that simultaneously allow the coexpression of two proteins of interest, the recovery of these proteins by protease treatment, and their subsequent purification by affinity chromatography. The plasmids developed will enable the co-transformation of plasmids in a single yeast because they contain different selection markers. This possibility of co-transformation increases the applications of the YDS system. On the one hand, in biocatalysis applications, it allows the introduction of more than one protein on the surface. Other similar systems are based on introducing anchor sites in AGA2 and the external production of proteins that recognize these sites, but this greatly complicates the efficiency and reproducibility of the reactions. In library screening experiments, it is also an advantage because the representativeness of the library can be doubled so that each yeast would contain the information for two surface-exposed proteins.

Based on the above, the inventors have developed new elements, such as the above-mentioned yeasts and plasmids, which allow the optimization of the YSD system. These will be described in detail below, along with their particular embodiments.

### Yeast of the invention, its uses, and method of production

As explained above, the developed yeasts allow the optimization of the surface exposure of the protein of interest, thus overcoming the drawbacks of other existing methods in the state of the art.

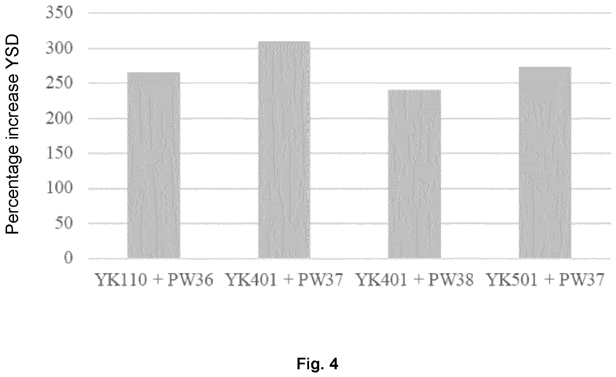

Therefore, in a first aspect, the present invention relates to a recombinant yeast, hereinafter "yeast of the invention", characterized in that its genome comprises a metabolite-inducible promoter instead of the native promoter of the AGA1 gene. The inventors have replaced the native promoter regulating the AGA1 gene with a metabolite-inducible promoter. In this way, AGA1 protein expression on the yeast surface only occurs in the presence of the inducing metabolite. Without induction, the yeast surface lacks the natural AGA1-AGA2 complex since AGA1 is not being expressed, and even if natural AGA2 does exist, the complex will not form on the surface. With this strategy, the formation of natural AGA1-AGA2 complexes that would compete with the YSD complexes designed in the present invention is avoided, and therefore the efficiency of the coating with the constructs described here is increased. This aspect was tested by flow cytometry with the three yeast strains used in the examples (yK110, yK401, and yK501), demonstrating an increase of exposure around 300% in yK401, and between 150- 250% in yK110 and yK501 (see Examples and Fig. 4).

The yeast of the invention may be, for example, any yeast of the genus Saccharomyces ssp. , such as, but not limited to,, *Saccharomyces cerevisiae, S. arboricolus, S. bayanus, S. bulderi S. cariocanus. S. cariocus, S. cerevisiae, S. cerevisiae var. boulardii, S. chevalieri, S. dairenensis, S. ellipsoideus, S. eubayanus. S. exiguus, S. florentinus, S. fragilis, S. kudriavzevii, S. martiniae, S. mikatae, S. monacensis, S. norbensis, S. paradoxus, S. pastorianus, S. spencerorum, S. turicensis, S. unisporus, S. uvarum, y S. zonatus.* However, in another particular embodiment, the yeast of the invention is Saccharomyces cerevisiae. Examples of *S*. *cerevisiae* species yeasts include, but are not limited to, BY4741, W303, Fm17, M2n, MEL2, HR4 WL3, YI30, KA31, and AH22.

In the yeast of the invention, the native promoter of the AGA1 gene has been replaced in the genome by a metabolite-inducible promoter. Genetic engineering techniques that allow the manipulation of the genome and the replacement of one part of the genome by another are widely known in the state of the art and any of them can be used in the context of the present invention. Thus, examples of such techniques include, but are not limited to, homologous recombination, integrase-mediated specific recombination, and nucleases such as the CRISP/Cas system or "transcription activator-like activity nuclease" or TALEN. Transcription activator-like effector nuclease).

The AGA1 gene (Descriptive name: a-agglutinin; Systematic name: YNR044W; SGD ID: SG-S000005327; Saccharomyces Genomic Database) codes for the Agap1 protein, being this Agap1 protein the anchoring subunit of a-agglutinin in cells; It is a highly O-glycosylated protein with a secretion signal at the N-terminal end, and with a region for GPI anchoring to the cell wall at the C-terminal end, linked to the Aga2p adhesion subunit through two disulfide bridges. The AGA1 gene comprises the nucleotide sequence SEQ ID NO: 20 (AGA1 YNR044W SGDID:S000005327, chrXIV:703699..705876).

SEQ ID NO: 20.
ATGACATTATCTTTCGCTCATTTTACCTACCTGTTCACAATATTGTTGGGATTAACTAATATTG
CCTTGGCATCTGATCCAGAAACGATTCTAGTGACGATAACCAAGACAAACGATGCAAATGGGGT
TGTTACAACTACAGTTTCACCCGCGCTAGTCTCCACATCCACTATCGTTCAAGCTGGCACTACG
ACATTGTATACGACTTGGTGTCCATTGACGGTATCCACTTCATCTGCTGCCGAAATAAGTCCTT
CAATATCGTACGCTACTACCCTATCCAGATTTAGTACTTTGACATTATCTACAGAAGTCTGCTC
CCATGAGGCATGTCCTTCGTCATCGACGTTGCCAACCACCACCTTATCTGTGACTTCCAAGTTC
ACTTCATATATTTGCCCTACTTGTCACACAACCGCTATCAGCTCATTATCCGAAGTAGGAACTA
CAACCGTGGTATCATCCAGCGCCATTGAACCATCAAGTGCCTCTATAATCTCACCTGTCACCTC
TACACTTTCGAGTACAACATCGTCCAATCCAACTACTACCTCCCTAAGTTCGACATCTACATCT
CCAAGCTCTACATCTACATCTCCAAGCTCTACATCTACCTCATCAAGTTCGACATCTACCTCAT
CAAGTTCGACATCTACCTCATCAAGTTCGACATCTACATCTCCAAGTTCGACATCCACATCTTC
AAGTTTGACATCCACATCTTCAAGTTCTACATCTACATCCCAAAGTTCTACATCTACCTCATCA
AGTTCGACATCTACATCTCCAAGCTCTACATCTACCTCATCAAGTTCAACATCTACATCTCCAA
GTTCTAAATCTACTTCTGCAAGCTCCACTTCCACTTCTTCATATTCAACATCTACATCCCCAAG
TTTGACTTCTTCATCTCCAACTTTGGCTTCCACTTCTCCAAGTTCAACATCTATTAGCTCTACT
TTTACTGATTCAACTTCATCCCTTGGCTCCTCTATAGCATCTTCATCAACGTCTGTGTCATTAT
ACAGCCCATCCACACCTGTTTACTCCGTCCCTTCGACTTCGTCAAATGTTGCAACTCCTTCTAT
GACTTCTTCAACTGTTGAAACAACTGTTAGTTCACAAAGTTCGTCTGAATATATCACCAAATCC
TCAATTTCTACTACTATCCCATCATTTTCCATGTCTACATATTTCACCACTGTTAGTGGAGTCA
CTACAATGTATACGACATGGTGTCCTTATAGCTCTGAATCTGAGACTAGCACATTAACCAGTAT
GCATGAAACGGTTACAACAGACGCTACAGTCTGCACTCACGAGTCTTGCATGCCCTCGCAGACA

ACAAGTTTGATTACATCTTCTATAAAAATGTCCACTAAAAACGTCGCAACTTCTGTAAGCACCT
CAACGGTTGAATCCTCATATGCATGCTCCACATGTGCTGAAACGTCACACTCGTATTCTTCCGT
GCAAACAGCTTCATCAAGTTCTGTAACACAGCAGACCACATCCACAAAGAGTTGGGTAAGTTCA
ATGACAACTTCGGATGAAGATTTCAATAAGCACGCTACCGGTAAGTATCATGTAACATCTTCAG
GTACCTCAACCATTTCGACTAGTGTAAGTGAAGCCACGAGTACATCAAGCATTGACTCAGAATC
TCAAGAACAATCATCACACTTATTATCGACATCGGTCCTTTCATCCTCCTCCTTGTCTGCTACA
TTATCCTCTGACAGTACTATTTTGCTATTCAGTTCTGTATCATCACTAAGTGTCGAACAGTCAC
CAGTTACCACACTTCAAATTTCTTCAACATCAGAGATTTTACAACCCACTTCTTCCACAGCTAT
TGCTACAATATCTGCCTCTACATCATCACTTTCCGCAACATCTATCTCTACACCATCTACCTCT
GTGGAATCGACTATTGAATCTTCATCATTGACTCCGACGGTATCTTCTATTTTCCTCTCATCAT
CATCTGCTCCCTCTTCTCTACAAACATCTGTTACCACTACAGAAGTTTCCACTACTTCAATCTC
CATACAATACCAAACTTCATCAATGGTAACAATTAGCCAATATATGGGCAGTGGATCGCAAACG
CGTTTGCCATTAGGAAAGTTGGTCTTCGCCATCATGGCAGTTGCTTGCAATGTAATTTTCAGTT
AA

In the yeast of the invention, the native promoter of the AGA1 gene is replaced by a metabolite-inducible promoter. In the present invention, the term "promoter" or "promoter sequence" refers to a nucleotide sequence located in the 5' direction of the transcriptional start of a gene, involved in the recognition and binding of RNA polymerase and other proteins responsible for the transcription of a gene in the 3' direction. Likewise, in the present invention, an "inducible promoter" is one that has initiated or increased the initiation of transcription of the regulated gene in response to a chemical, environmental or physical stimulus. Examples of inducible promoters include, but are not limited to, galactose inducible promoter (PGAL1); copper inducible promoter (PCUP1), tetracycline regulatory system (Tet-on and Tet-off), cyanamide inducible promoter (PDDI2). All these promoters are known to the person skilled in the art and are commercially available. In a particular embodiment of the yeast of the invention, the metabolite-inducible promoter is the galactose-inducible promoter. In another more particular embodiment of the yeast of the invention, the nucleotide sequence of the galactose-inducible promoter comprises, or consists of, the sequence SEQ ID NO: 1.

SEQ ID NO: 1.
TCATTATCAATACTCGCCATTTCAAAGAATACGTAAATAATTAATAGTAGTGATTTTCCTAACT
TTATTTAGTCAAAAAATTAGCCTTTTAATTCTGCTGTAACCCGTACATGCCCAAAATAGGGGGC
GGGTTACACAGAATATATAACATCGTAGGTGTCTGGGTGAACAGTTTATTCCTGGCATCCACTA
AATATAATGGAGCCCGCTTTTTAAGCTGGCATCCAGAAAAAAAAAGAATCCCAGCACCAAAATA

TTGTTTTCTTCACCAACCATCAGTTCATAGGTCCATTCTCTTAGCGCAACTACAGAGAACAGGG
GCACAAACAGGCAAAAAACGGGCACAACCTCAATGGAGTGATGCAACCTGCCTGGAGTAAATGA
TGACACAAGGCAATTGACCCACGCATGTATCTATCTCATTTTCTTACACCTTCTATTACCTTCT
GCTCTCTCTGATTTGGAAAAAGCTGAAAAAAAAGGTTGAAACCAGTTCCCTGAAATTATTCCCC
TACTTGACTAATAAGTATATAAAGACGGTAGGTATTGATTGTAATTCTGTAAATCTATTTCTTA
AACTTCTTAAATTCTACTTTTATAGTTAGTCTTTTTTTTAGTTTTAAAACACCAGAACTTAGTT
TCGA

The YSD technique comprises the exposure of a protein of interest on the yeast surface. For this purpose, it is necessary to express the protein of interest within yeast. Thus, to express the protein of interest inside the yeast of the invention, a set of plasmids have been designed with a series of characteristics that allow not only to express the protein of interest, but also to enable co-transformation with other plasmids being able to express two proteins of interest simultaneously, the recovery of the protein exposed on the surface of the yeast, and the purification of the recovered protein.

Therefore, in another particular embodiment, the yeast of the invention is characterized in that it further comprises one or more plasmids, wherein each plasmid comprises:
(i) a metabolite-inducible promoter operatively linked to the nucleotide sequence coding for a fusion protein, wherein fusion protein comprises in N-terminal - C-terminal direction: (a) the amino-terminal region of the Aga2 protein, (b) a first tag for immunological detection, (c) a protein of interest, (d) a second tag for immunological detection, and (e) a tag sequence for the protein of interest purification;
(ii) a broad-spectrum promoter operationally linked to a selection marker; and
(iii) a CEN/ARS centromeric origin of replication.

In another still more particular realization, the plasmid comprised within the yeast of the invention includes, between components (b) and (c), an endopeptidase recognition sequence.

In another preferred embodiment, the amino-terminal region of the Aga2 protein is the sequence comprising or consisting of SEQ ID NO: 31.

SEQ ID NO: 31:
MQLLRCFSIFSVIASVLAQELTTICEQIPSPTLESTPYSLSTTTILANGKAMQGVFEYYKS
VTFVSNCGSHPSTTSKGSPINTQYVF

In another particular embodiment of the yeast of the invention, the first tag for immunological detection is an HA epitope.

In another more particular embodiment of the inventive yeast, the plasmid further comprises a protease recognition site preferably recognized by TEV protease or THR protease.

In another particular embodiment of the yeast of the invention, the protein of interest is selected from the list consisting of:
- Antibodies in all their versions, or fragments thereof, including human antibodies (Wei Sun et al. Acta Pharm Sin B. 2019;9(5):960-972.), or reduced versions thereof, such as scFv (Chao G et al. Nat Protoc. 2006; 1(2):755-68).
- Enzymes with degradative activity, such as cellulases and hemicellulases, used in lignocellulosic biomass degradation and ethanol production (Tabañag I.D.F et al. Catalysts. 2018;8(3):94).
- Proteins absorbing toxins or food contaminations, such as Silicateins (Hongying Wang et al. ACS Omega. 2020; 5(13): 7555-7566).
- Enzymes with electronic acceptor or donor capability for biocatalysis chemical processes or biosensors (Pham ML. Polakovič M. Int J Biol Macromol. 2020;15(165) 835-841).

In another particular embodiment of the yeast of the invention, the second immunological detection tag is a c-Myc epitope.

In another particular embodiment of the yeast of the invention, the marker is an auxotrophic marker. In yet another more particular embodiment, the auxotrophic marker is TRP, HIS, or LEU.

In another particular embodiment of the yeast of the invention, the purification tag of the protein of interest is a histidine tail.

In another particular embodiment of the yeast of the invention, the plasmid comprises the nucleotide sequence SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

In another particular embodiment of the yeast of the invention, the yeast is characterized in that it comprises two plasmids selected from the list consisting of SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

In another particular embodiment of the yeast of the invention, one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

In another particular embodiment of the yeast of the invention, one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

In the present invention, all the particular embodiments described for the yeast of the invention are contemplated alone or in combination with all of the above. Likewise, particular embodiments referring to the plasmid comprised within the yeast of the invention will be described in detail below, in which the plasmid is considered another inventive aspect of the present invention, referring to it as "the plasmid of the invention".

As the person skilled in the art understands, the yeast of the invention may be forming part of a composition. Thus, in another aspect, the present invention relates to a composition comprising the yeast of the invention.

The composition described herein may be a liquid or solid composition, and must comprise all those compounds or nutrients that allow the growth, maintenance and/or development of the yeast of the invention. In the context of the present invention, the culture medium in which the yeast grows and develops is considered composition. Compositions or culture media useful in the maintenance, growth, and development of yeasts are widely known to the person skilled in the art and are commercially available.

General yeast growth media contain an energy source, such as carbohydrates, and agar. Yeasts grow at room temperature, with an oxygen pressure of 1 atmosphere and at a slightly acidic pH, which can help to keep bacteria from growing in this rich medium. To maintain extrachromosomal material in plasmid form, antibiotics are sometimes added to yeast media, often gentamicin, kanamycin, or chloramphenicol, that inhibit the growth of non-plasmid yeast. Positive selection of plasmids can also be made by complementation of amino acid biosynthesis pathways. The plasmid contains the information necessary to complete an amino acid synthesis pathway (such as leucine, tryptophan, histidine, etc.) that is interrupted in the recipient strain. In this case, the culture medium is preferably a minimal medium filled with all amino acids except the one used for selection. In the sense of the present invention, a "minimal medium" means an aqueous culture medium containing all substrates (e.g., carbon source, mineral salts, trace elements, vitamins) necessary for the growth of yeast cells in dissolved form, but which at the same time does not contain complex constituent substances or complex biomolecules (e.g., yeast extract, peptone, tryptone, etc.). The skilled person in the art knows a large number of minimal media for growing yeast cells. Some common and general yeast growth media have an acidic pH such as Sabouraud Agar or potato dextrose agar. The most complete medium on which yeast can be grown and the one commonly used to grow *S*. *cerevisiae* in the laboratory is YEPD or YPD. It includes yeast extract, usually 1% mass/volume ratio with water, 2% peptone, 2% glucose or dextrose as an energy source, and double distilled water to the desired volume. If a solid medium is to be made, agar is added at the desired concentration, being the most commonly 1 or 1.5% agar.

In another aspect, the present invention relates to a yeast library, hereinafter "yeast library of the invention", comprising one or more yeasts of the invention. The yeast libraries could be synthetic antibody libraries, whether or not they have been generated from animal material. In the case of proteins with enzymatic activity, the libraries may contain several versions of the same enzyme, obtained either from described sequences or through directed evolution methods. Libraries are generated either from synthetic DNA or from natural libraries in phages, plasmids, cosmids, etc. obtained from genomic material or cDNAs. As described above, each of these yeasts could express up to two proteins of interest.

The present invention also contemplates the use of the yeast of the invention in the yeast surface exposure technique or YSD. This technique is widely known in state of the art and can be consulted in textbooks and laboratory manuals, its implementation being routine laboratory practice for the person skilled in the art. Thus, in another aspect, the invention relates to the use of the yeast of the invention in the YSD technique.

The method of obtaining the yeast of the invention constitutes another aspect of the present invention. Therefore, the invention also relates to a method for obtaining the yeast of the invention, hereinafter "first method of the invention", comprising a step (a) comprising genetic replacement by homologous recombination of the natural promoter of the *AGA1* gene in yeast by a metabolite-induced promoter.

The *AGA1* gene as well as the promoter regulating it, have been described in previous paragraphs, and both their explanation and their particular embodiments apply to the present inventive aspect. Similarly, metabolite-inducible promoters have also been described in the previous inventive aspect and both their definition and their particular embodiments are applicable to the present inventive aspect. Thus, in another particular embodiment of the first method of the invention, the metabolite-inducible promoter is the galactose-inducible promoter, wherein, in yet another more particular embodiment, the nucleotide sequence of the galactose-inducible promoter comprises or consists of the sequence SEQ ID NO: 1.

The yeast of the invention may be any yeast of the genus *Saccharomyces* ssp. Such as, but not limited to, *Saccharomyces cerevisiae, S. arboricolus, S. bayanus, S. bulderi S. cariocanus. S. cariocus, S. cerevisiae, S. cerevisiae var. Boulardii, S. chevalieri, S. dairenensis, S. ellipsoideus, S. eubayanus. S. exiguus, S. florentinus, S. fragilis, S. kudriavzevii, S. martiniae, S. mikatae, S. monacensis, S. norbensis, S. paradoxus, S. pastorianus, S. spencerorum, S. turicensis, S. unisporus, S. uvarum,* and *S*. *zonatus.* However, in another particular embodiment, the yeast of the invention is *Saccharomyces cerevisiae.* Examples of yeasts of the species *S. cerevisiae* include, but are not limited to, BY4741, W303, Fm17, M2n, MEL2, HR4 WL3, YI30, KA31, and AH22.

In another particular embodiment of the first method of the invention, the method further comprises a step (b) comprising transforming the yeast obtained in step (a) with one or more plasmids, wherein each plasmid contains (i) a metabolite-inducible promoter operably linked to the nucleotide sequence coding for a fusion protein, wherein said fusion protein comprises in N-terminal - C-terminal direction: (a) the amino-terminal region of the Aga2 protein, (b) a first tag for immunological detection, (c) a protein of interest, (d) a second tag for immunological detection, and (e) a tag for purification of the protein of interest; (ii) a broad-range promoter functionally linked to a selection marker, and (iii) a CEN/ARS centromeric origin of replication.

In another still more particular embodiment of the first method of the invention, the plasmid referred to in the preceding paragraph comprises, between components (b) and (c), an endopeptidase recognition sequence.

In another preferred embodiment, the amino-terminal region of the Aga2 protein is the sequence comprising or consisting of SEQ ID NO: 31.

In another particular embodiment of the first method of the invention, the first immunological detection tag is an HA epitope.

In another particular embodiment of the first method of the invention, the plasmid further comprises a protease recognition site preferably recognized by TEV protease or THR protease.

In another particular embodiment of the first method of the invention, the protein of interest is selected from the list consisting of antibodies or antibody fragments, enzymes with degradative activity, proteins absorbing toxins or food contaminations, and enzymes with acceptor or electron-donating capacity for biocatalysis chemical processes.

In another particular embodiment of the first method of the invention, the second immunological detection tag is a c-Myc epitope.

In another particular embodiment of the first method of the invention, the marker is an auxotrophic marker where, in yet another more particular embodiment, the auxotrophic marker is TRP, HIS or LEU.

In another particular embodiment of the first method of the invention, the purification tag of the protein of interest is a histidine tail.

In another particular embodiment of the first method of the invention, the plasmid(s) of step (b) is/are selected from the list consisting of SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

In another more particular embodiment of the first method of the invention, and in the case where the yeast is transformed with two plasmids, one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

Alternatively to the above particular embodiment, in another particular embodiment of the first method of the invention, and in the case where the yeast is transformed with two plasmids, one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

In the present invention, all the particular embodiments described for the first method of the invention are contemplated alone or in combination with all of the above. Likewise, the particular embodiments referring to the plasmid employed in the first method of the invention will be described in detail below since said plasmid is considered another inventive aspect of the present invention, referring to it as "the plasmid of the invention".

### Plasmid of the invention and its uses

As described at the beginning of the present description, the inventors have developed a set of plasmids that simultaneously allow the co-expression of two proteins of interest, the recovery of said proteins by treatment with proteases, and their subsequent purification by affinity chromatography. The plasmids thus developed allow co-transformation of the plasmids in a single yeast because they contain different selection markers. This possibility of co-transformation increases the applications of the YDS system. On the one hand, it can be used in biocatalysis applications since it allows the introduction of more than one protein on the surface. Other similar systems are based on the introduction of anchor sites in AGA2 and the external production of proteins that recognize these sites, but this greatly complicates the efficiency and reproducibility of the reactions. In library screening experiments, it is also an advantage because the representativeness of the library can be doubled so that each yeast would contain the information for two surface-exposed proteins. The plasmids developed by the inventors in the present invention will be described in detail below, together with all their particular embodiments.

Thus, in another aspect, the present invention relates to a plasmid, hereinafter "plasmid of the invention", comprising:
(i) a metabolite-inducible promoter operably linked to the nucleotide sequence coding for a fusion protein, wherein said fusion protein comprises in the N-terminal - C-terminal direction: (a) the amino-terminal region of the Aga2 protein, (b) a first tag for immunological detection, (c) a protein of interest, (d) a second tag for immunological detection, and (e) a purification tag of the protein of interest;
(ii) a broad-range promoter functionally linked to a selection marker; and
(iii) a CEN/ARS centromeric origin of replication.

In the present invention, "plasmid" means a circular or linear extrachromosomal DNA molecule that replicates and transcribes independently of chromosomal DNA. As used in the present invention, the terms "plasmid" and "vector" (with self-replicating capacity) are considered equivalent and may be used interchangeably.

In the present invention, the plasmid is employed to introduce and express a specific gene in a cell of interest, particularly in a yeast. Therefore, the plasmid of the invention is considered an expression vector. Expression vectors allow the production of large amounts of stable messenger RNA (mRNA). The proteins encoded by the plasmid of the invention are produced by the transcription and translation machinery of the cell once the vector is inside the cell. In a particular embodiment, the plasmid is a broad host-spectrum plasmid, i.e., a plasmid comprising the genetic elements necessary to be functional in multiple host cells.

In another still more particular embodiment, the plasmid of the invention comprises, between components (b) and (c), an endopeptidase recognition sequence.

In another preferred embodiment, the amino-terminal region of the Aga2 protein is the sequence comprising or consisting of SEQ ID NO: 31

The plasmid of the invention comprises a first immunological detection tag. In the present invention, "tag for immunological detection" means a molecule, or epitope, expressly recognized by an antibody so that immunological techniques can detect the recombinant protein produced and comprising such epitope. Such tags are widely known in the prior art and are commercially available. Examples of Tags include but are not limited to c-Myc (originating from the proto-oncogene c-myc), DYKDDDDDDK tag (also called Sigma's FLAG^{®} tag, SEQ ID NO: 32), GFP (*Green fluorescent protein*)*,* Glu-Glu (CEEEEYMPME tag, SEQ ID NO: 33, originally isolated from polyoma virus median T antigen), GST (*glutathione S-transferase*)*,* HA (hemagglutinin), 6xHIS (polyhistidine or Hexa-histidine tag), mCherry (fluorescent protein), S-Tag (epitope derived from the first 15 amino acids of the amino-terminal end of pancreatic ribonuclease A), Strep-Tag (8 amino acid epitope with sequence WRHPQFGG, SEQ ID NO: 34), T7 (11 amino acid long peptide encoded from the leader sequence of bacteriophage T7 gene 10), thioredoxin, V5 (epitope derived from the alpha subunit of simian parainfluenza virus type 5 RNA polymerase), and VSV-G (epitope derived from vesicular stomatitis virus, SEQ ID NO: 35). In a particular embodiment of the plasmid of the invention, the first immunological detection tag is an HA epitope.

On the other hand, the plasmid of the invention also comprises a protease recognition site. This site allows the protein of interest produced to be cleaved or separated from the other components of the recombinant protein and recovered by treatment with proteases for subsequent *in vitro* assays. Examples of proteases employed for this purpose include, but are not limited to, TEV protease (cleavage sequence is ENLYFQG, SEQ ID NO: 36), HRV3C protease (cleavage sequence LFQGP, SEQ ID NO: 37), Thrombin protease (excision sequence LVPRGS, SEQ ID NO: 38), FactorXA protease (excision sequence I(E/D)GR, SEQ ID NO: 39) and enterokinase protease (excision sequence DDDDK, SEQ ID NO: 40). In a particular embodiment of the plasmid of the invention, the protease recognition site is recognized by TEV protease or THROMBIN protease (THR).

The plasmid of the invention also comprises a protein of interest. Examples of proteins of interest include, but are not limited to, antibodies or antibody fragments (SdAb, scFv, etc...), enzymes with degradative activity, toxin or food contamination absorbing proteins, and enzymes with acceptor or electron-donating capacity for biocatalysis chemical processes.

Another of the plasmid elements of the invention is a second immune detection tag. The term "tag for immunological detection" has been defined previously, as well as examples thereof. The integrity of the surface-exposed protein can be checked thanks to the second tag for immunological detection. In another particular embodiment of the plasmid of the invention, the second tag for immunological detection is a c-Myc epitope.

In another particular embodiment, the plasmid of the invention also comprises at its c-terminal end a nucleotide sequence encoding a histidine tail that allows purification of the protein of interest once cleaved. As in the skilled person in the art understands, any nucleotide sequence encoding a purification tag can be employed in the context of the present invention. Examples of purification tags include, without limitation, His-tag (polyhistidine), GST Tag (Glutathione S-transferase), MBP Tag (maltose binding protein), CBP Tag (calmodulin binding peptide), and streptavidin/Biotin-based tag [BCCP(biotinylation signal peptide)+biotin].

The plasmid of the invention also comprises a nucleotide sequence encoding a selection marker. This selection marker makes it possible to identify the cell comprising the plasmid of the invention as opposed to others that have not incorporated it. In the present invention, a "selection marker", "selection marker gene," or "indicator gene" includes any gene that confers a phenotype on a cell in which it is expressed to facilitate the identification and/or selection of cells that are transfected with a nucleotide sequence or plasmid as described herein. Suitable markers may be selected from markers that confer antibiotic resistance, introduce a novel metabolic trait, or allow visual selection. Examples of selection marker genes include genes that confer antibiotic resistance (such as nptl, which phosphorylates neomycin and kanamycin, or hpt, which phosphorylates hygromycin, or genes that confer resistance to, for example, bleomycin, streptomycin, tetracycline, chloramphenicol, ampicillin, gentamicin, geneticin (G418), spectinomycin or blasticidin), or genes that provide a metabolic trait. Expression of visual marker genes results in the formation of color (e.g., 13-glucuronidase, GUS, or 13-galactosidase with their color substrates, e.g., X-Gal), luminescence (such as the luciferin/luciferase system) or fluorescence (Green Fluorescent Protein, GFP, and derivatives thereof). This list represents only a small number of possible markers. The expert is familiar with these markers. Depending on the organism and the selection procedure, different markers are preferred. The genetic markers can be removed from the host cell if desired. In a particular embodiment, the selection marker is an auxotrophic marker. Any auxotrophic marker can be employed in the context of the present invention. Such markers are widely known to the person skilled in the art and are commercially available. Examples of auxotrophic markers include, without limitation, ADE1, ADE2, CAN1, HIS3, LEU2, LYS2, TRP1, TRP5, URA3, URA4, MET15. However, in a more particular embodiment of the plasmid of the invention, the auxotrophic marker is TRP, HIS, or LEU, more preferably TRP1, HIS3, or LEU2.

In another particular embodiment of the plasmid of the invention, the plasmid comprises the nucleotide sequence SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

As explained at the beginning of this invention, the plasmid of the invention is designed so that it can be co-transformed with other plasmids so that the cell, particularly a yeast, more particularly the yeast of the invention, can express two proteins of interest simultaneously. Thus, in another aspect, the present invention relates to a plasmid pair, hereinafter "plasmid pair of the invention", wherein the plasmid pair is selected from the plasmid listing consisting of the sequences SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

In a particular embodiment of the plasmid pair of the invention, one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

In another particular embodiment of the plasmid pair of the invention, one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

Both the plasmid and the plasmid pair of the invention can be introduced into a cell. Thus, in another aspect, the invention relates to a host cell, hereinafter "host cell of the invention", comprising the plasmid of the invention or the plasmid pair of the invention. Examples of host cells suitable for harboring the plasmid or plasmid pair of the invention include, without limitation, mammalian, plant, insect, yeast/fungal, fungal, and bacterial cells. Bacterial cells include, without limiting, Gram-positive bacterial cells such as species of the genus *Bacillus, Streptomyces* and *Staphylococcus* and Gram-negative bacterial cells such as cells of the genus *Escherichia* and *Pseudomonas.* Insect cells include but are not limited to, Drosophila cells and Sf9 cells. Plant cells include, but are not limited to, cells from crop plants such as cereals, medicinal, ornamental, or bulb plants. Mammalian cells suitable for the present invention include epithelial cell lines (porcine, etc.), osteosarcoma cell lines (human, etc.), neuroblastoma cell lines (human, etc.), epithelial carcinomas (human, etc.), glial cells (murine, etc.), liver cell lines (monkey, etc.), and CHO (Chinese Hamster Ovary) cells. In a particular embodiment of the host cell of the invention, the host cell is a yeast cell. Yeast cells include, but are not limited to, yeasts of the genus *Saccharomyces, Kluyveromyces, Pichia, Candida, and Yarrowia,* any of which may be used as host cells of the invention. In a more particular embodiment, the host cell is *Saccharomyces cerevisiae, Yarrowia lipolytica, Schizosaccharomyces pombe, Kluyveromyces lactis, Kluyveromyces marxianus, Candida lipolytica, Torulopsis glabrata, Rhodotorula glutinis, Rhodotorula graminis, Saccharomyces pastorianus, Candida tropicalis, Zygosaccharomyces rouxii, Candida glabrata, Torulaspora delbrueckii, Debaryomyces hansenii, Scheffersomyces stipites, Meyerozyma guilliermondii, Lodderomyces elongisporus, Candida albicans, Candida orthopsilosis, Candida metapsilosis, Candida dubliniensis, Clavispora lusitaniae, or Candida auris.* However, in another, still more particular embodiment, the host cell of the invention is *Saccharomyces cerevisiae.* Examples of yeasts of the species *S*. *cerevisiae* include, but are not limited to, BY4741, W303, Fm17, M2n, MEL2, HR4 WL3, YI30, KA31 and AH22.

Preferably, this host cell of the invention is deficient in proteases, a feature that improves the stability of the expressed fusion protein.

In another aspect, the present invention relates to a composition comprising the host cell of the invention.

In another aspect, the present invention relates to the use of the plasmid of the invention, or the plasmid pair of the invention, to transform or co-transform a host cell, preferably a yeast, in particular, the yeast of the invention.

Methods for introducing gene constructs, vectors or plasmids into a cell are widely known to the skilled person in the art and their use is standard laboratory practice. The term "introduce" in the context of the present invention refers to the incorporation of a nucleotide sequence into a cell by "transfection" or "transformation" or "transduction", wherein the nucleotide sequence may be incorporated into the genome of the cell (e.g., chromosome, plasmid, mitochondrial DNA), or be a stand-alone replicon, or be transiently expressed (e.g., transfected mRNA). As used herein, the terms "transformed," "stably transformed," or "transgenic," with reference to a cell, mean that the cell has a non-native (heterologous) nucleotide sequence integrated into its genome or into an episomal plasmid that is maintained across multiple generations.

Examples of methods for introducing gene constructs, vectors, or plasmids into host cells include, but are not limited to, electroporation; nuclear microinjection or direct microinjection into single cells; fusion of bacterial protoplasts with intact cells; use of polycations, e.g., polybrene or polyornithine; membrane fusion with liposomes, lipofectamine-mediated transfection, or lipofection; high-speed bombardment with DNA-coated microprojectiles; incubation with calcium phosphate-DNA precipitation; DEAE-dextran-mediated transfection; infection with modified viral nucleic acids; Agrobacterium-mediated DNA transfer and the like.

### YSD method of the invention

In another aspect, the present invention relates to a method for exposing a protein of interest on the surface of a yeast, hereinafter, "second method of the invention", comprising:
(i) transform the yeast of the invention with one or more plasmids of the invention, or with a pair of plasmids of the invention, and
(ii) grow the yeast transformed in step (i) under conditions suitable for the expression of the protein(s) of interest on the yeast surface.

Both the yeast and the plasmid or plasmid pair of the invention have been described in previous inventive aspects and both their definitions and their particular embodiments are applicable to the present inventive aspect.

In a first stage [stage (i)], the second method of the invention includes transforming the yeast of the invention with one or more plasmids of the invention, or with a pair of plasmids of the invention. In the present inventive aspect, the term "transform" is understood to mean a procedure comprising introducing a nucleotide sequence into the yeast of the invention. Examples of methods for introducing plasmids, into yeast include, without limitation, chemical transformation with LiAc and PEG, electroporation, nuclear microinjection or direct microinjection into single cells; the fusion of bacterial protoplasts with intact cells; use of polycations, e.g., polybrene or polyornithine; membrane fusion with liposomes, lipofectamine-mediated transfection, or lipofection; high-speed bombardment with DNA-coated microprojectiles; incubation with DNA-calcium phosphate precipitation; DEAE-dextran-mediated transfection; infection with modified viral nucleic acids; and the like. In a particular embodiment of the second method of the invention, transformation of the yeast in step (i) is carried out by chemical transformation with LiAc and PEG.

In the second stage [stage (ii)], the second method of the invention comprises culturing the yeast transformed in stage (i) under conditions suitable for the expression of the protein(s) of interest on the yeast surface. Conditions and culture media for culturing yeast have been explained in a previous inventive aspect, namely, for the yeast of the invention, and such media and conditions are applicable to the second method of the invention.

After step (ii) of the second method of the invention, a yeast is obtained which comprises on its surface the protein of interest introduced with the plasmid of the invention. Examples of proteins of interest have been cited previously. Thus, in a particular embodiment of the second method of the invention, the protein of interest is selected from the list consisting of antibodies or antibody fragments (SdAb, scFv, etc...), enzymes with degradative activity, proteins absorbing toxins or food contaminations, enzymes with acceptor or electron-donating capacity for biocatalysis chemical processes.

### Kit of the invention and its uses

The implementation of the invention comprises the use of the cell, the yeast, the plasmid of the invention, etc., which may form part of a kit.

Therefore, in another aspect, the present invention relates to a kit, hereinafter the "kit of the invention", comprising:
- the yeast of the invention, and/or
- the composition of the invention, and/or
- the yeast library of the invention, and/or
- the plasmid of the invention, and/or
- the plasmid pair of the invention, and/or
- the host cell of the invention.

All of the components of the kit have been defined and explained in previous paragraphs of the present description, as well as their particular embodiments, all of which are applicable to the kit of the invention.

In addition to one or more of the above-mentioned elements, the kit may comprise other components useful in the implementation of the present invention, such as, buffers, delivery vehicles, material supports, positive and/or negative control components, etc. Likewise, the kits may also include instructions for practicing the object of the invention. These instructions may be present in said kits in a variety of forms, one or more of which may be present in the kit. One form in which these instructions may be present is as printed information on a suitable medium or substrate, e.g., a sheet or sheets of paper on which the information is printed, on the kit packaging, on a package insert, etc. Another medium would be a computer readable medium, e.g., a CD, USB, etc., on which the information has been recorded. Another medium that may be present is a website address that can be used over the Internet to access the information at a remote site. Any convenient means may be present in the kits.

On the other hand, the present invention also relates to the use of the kit of the invention in a method for exposing a protein of interest on the surface of a yeast, or in a method for transforming a cell, preferably, a yeast, or in the yeast surface display technique, or in a method according to the first method of the invention described herein.

In a particular embodiment of the kit of the invention, the yeast is the yeast of the invention.

The details of these uses have already been described in previous paragraphs of the present description.

### DESCRIPTION OF THE FIGURES

**Figure 1****.** Promoter replacement method used to generate the yK110, yK401, and yK501 strains. A fragment was generated by PCR with the auxotrophic complementation selection marker with URA3 and flanked with homologous sequences for recombination with the natural AGA1 promoter.
**Figure 2****.** Schematic of the PW series plasmid constructs. A- Construction with a TEV or THR protease recognition site. B-Construct with an N-terminal polyhistidine tag for affinity column purification.
**Figure 3****.** Representative map of the pW plasmids generated in the present invention.
**Figure 4****.** Percentage of cells that successfully express the yeast surface proteins containing the invention's plasmids versus the control strain EBY100.

### EXAMPLES

In the following, the invention will be illustrated by some tests carried out by the inventors, highlighting the usefulness of the yeast, the plasmid, and the methods described in the invention.

### I - MATERIALS and METHODS

### Yeast strains and culture conditions.

The Saccharomyces cerevisiae strains used in this study are shown in Table 1. Yeast strains were grown at 28 °C in a complete growth medium (YPD) containing 1 % yeast extract, 2 % bacterial peptone, and 2 % dextrose. Plasmid-transformed strains were grown in a synthetic growth medium (SD) containing 0.67 % yeast nitrogen base, 50 mm succinic acid, pH 3, and 2 % dextrose. Depending on the auxotrophies of each strain, adenine (0.025 g/liter), histidine (0.1 g/liter), leucine (0.1 g/liter), methionine (0.1 g/liter), tryptophan (0.05 g/liter) or uracil (0.025 g/liter) were added. In the case of GAL1-controlled promoter induction, galactose substituted dextrose at the same concentration.

**Table 1. Strains used in the invention.**

| **STRAIN** | **DESCRIPTION** | **ORIGIN** |
|---|---|---|
| BJ5465 | MATa ura3-52 trp1 leu2-deltal his3-delta200 pep4::HIS3 prbl-deltal.6R can1 GAL | ATCC |
| | | https://www.atcc.org |
| BY4741 pep4::KAN | MATa his3Δ1 leu2Δ0 met15Δ0 ura3Δ0 pep4::KAN | EUROSCARF |
| | | http://www.euroscarf.de |
| BY4741 prb1::KAN | MATa his3Δ1 leu2Δ0 met15Δ0 ura3Δ0 prb1::KAN | EUROSCARF |
| | | http://www.euroscarf.de |

### Construction of strains with improved display.

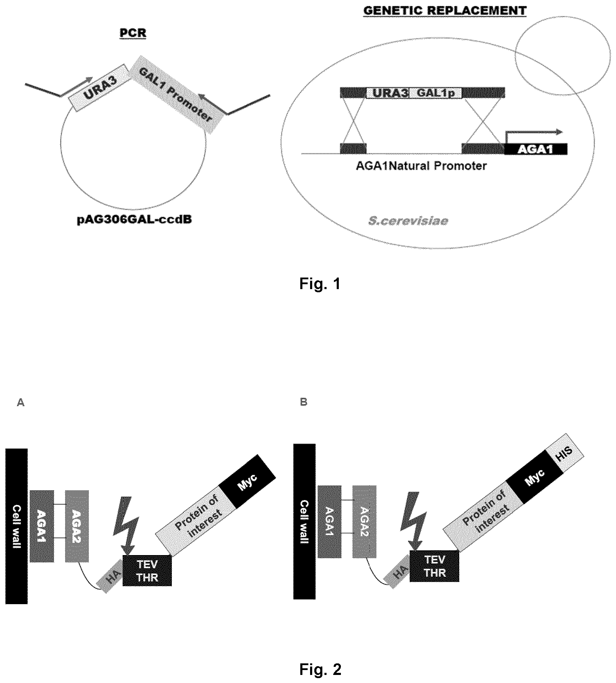

The strains were generated by gene replacement of the natural promoter of AGA1 gene so that, in all cases, its expression is controlled by a galactose-inducible promoter (Figure 1). The primers GAL1-AGA1 FW and GAL1-AGA1 RV were used (Table 2).

**Table 2. Design of primers used to replace the natural promoter of AGA1 with a Galactose-inducible promoter.**

| **Primers** | **SEQUENCE** |
|---|---|
| GAL1-AGA1 Fw | GGATATTGCACGTTTCAAGTCAAAACGCGGCTGTAACATAATTTTCGCGGCATCAG AGCAGATTGTACTGA (SEQ ID NO: 21) |
| GAL1-AGA1 Rv | CAATATTGTGAACAGGTAGGTAAAATGAGCGAAAGATAATGTCATGGGGGATCC ACTAGTTCTAGAATCCG (SEQ ID NO: 22) |

The region between the URA3 promoter and the GAL1 promoter was amplified by PCR using the oligonucleotides in Table 2 and the plasmid pAG306GAL-ccdB as the template (Alberti S. et al. Yeast. 2007;24(10):913-919). The primers contain homologous ends to those adjacent to the natural gene, so gene replacement replaced the natural promoter with an inducible one (Figure 1). The replacement was performed in three types of strains defective in protease systems; BJ5465, BY4741 pep4::KAN and BY4741 prb1::KAN. The transformation was performed by the LiAC/PEG chemical method (Gietz RD. et al. Nat Protoc. 2007;2(1):31-4). Cultures were grown overnight on shaking at 28°C until exponential growth in YPD, and 108 cells were used for chemical transformation, transformants were selected on synthetic medium plates without uracil.

### Plasmid construction.

Plasmids with different auxotrophies were generated by homologous recombination of plasmid pCTcon2 (ADDGENE https://www.addgene.org) with PCR products obtained from plasmids p415-GPD and p413-GPD (Mumberg D. et al. Gene. 1995 Apr 14;156(1):119-22). The specific protease recognition sites were inserted by oligonucleotide hybridization, digestion, and cloning on plasmids pCTcon2, PW22, and PW23, originating plasmids PW22-29. The addition of the 6 Histidines for purification was performed by oligonucleotide hybridization and homologous recombination with plasmid pCTcon2 and PW22-29, and originated plasmid PW30-38. All plasmids obtained were verified by sequencing. Table 3 shows the oligonucleotides used in the construction of the plasmids of the invention.

**Table 3 - Oligonucleotides used for the construction of the PW series plasmids.**

| **OLIGONUCLEOTIDES** | **SEQUENCE** |
|---|---|
| F1ori1-F | GTGGACTCTTGTTCCAAACTGG (SEQ ID NO: 23) |
| PRS-marker | CGGCATCAGAGCAGATTGTA (SEQ ID NO: 24) |
| TEV Fw | CCCCCTGCAGGAAAATTTGTATTTTCAATCTCTGCAGGGGG (SEQ ID NO: 25) |
| TEV Rev | CCCCCTGCAGAGATTGAAAATACAAATTTTCCTGCAGGGGG (SEQ ID NO: 26) |
| THROMBIN Fw | CCCCCTGCAGTTGGTTCCAAGAGGTTCTCTGCAGGGGG (SEQ ID NO: 27) |
| THROMBIN Rv | CCCCCTGCAGAGAACCTCTTGGAACCAACTGCAGGGGG (SEQ ID NO: 28) |
| HISF | GAACAAAAGCTTATTTCTGAAGAGGACTTGCACCACCACCACCACCACTGACTC GAGATCTGATAACAACAGTGTAGATGTAA (SEQ ID NO: 29) |
| HISR | TTACATCTACACTGTTGTTATCAGATCTCGAGTCAGTGGTGGTGGTGGTGGTGC AAGTCCTCTTCAGAAATAAGCTTTTGTTC (SEQ ID NO: 30) |

### Flow Cytometry

The PW plasmids obtained and tested were chemically transformed into the yeast strains of the invention and selected by growth on synthetic medium with the corresponding amino acids. Surface protein display was induced by growth with galactose for 24 hours at 28°C. Flow cytometry assays of approximately 150,000 yeast cells were performed using a Milteny MACSQuant flow cytometer, and using mouse anti-HA as the 1st antibody (Fisher,Ref 11558113.), and rabbit anti-mouse FITC as the 2nd antibody (Abcam, Ref.ab8517).

### II- RESULTS

### Obtaining the set of PW Plasmids

In most of the described works, constructs are obtained by modifications of plasmid pCTcon2 (with *TRP1* gene), which is used in strain EBY100 (MATa AGA1::GAL1-AGA1::URA3 ura3-52 trp1 leu2-delta200 his3-delta200 pep4::HIS3 prbd1.6R can1 GAL) (Boder ET, Wittrup KD. Nat Biotechnol. 1997;15(6):553-7).

**Table 5-Possible plasmid combinations for co-transformation in the S.c. strains generated in the invention.**

| **Strain** | **PLASMID 1** | **PLASMID 2** |
|---|---|---|
| YK110 | pCTcon2, PW24, PW27, PW30, PW33, PW36 | PW23, PW26, PW29, PW32, PW35, PW38 |
| YK401 | PW22, PW25, PW28, PW31, PW34, PW37 | PW23, PW26, PW29, PW32, PW35, PW38 |
| YK501 | PW22, PW25, PW28, PW31, PW34, PW37 | PW23, PW26, PW29, PW32, PW35, PW38 |

In addition, TEV and THR protease recognition sites have been introduced to recover the exposed protein. This modification is significant because it allows protein recovery by protease treatment for subsequent in vitro assays. The TEV and THR protease-dependent recovery system can be combined with different plasmids containing different selection markers and thus subsequently have the possibility of selective extraction of only one or both proteins.

Six HIS residues have also been introduced in the C-terminal region of the protein, allowing its purification. Other versions introduce these residues in the N-terminal part, but D Wittrup has already pointed out the need to control the complete expression of the proteins by means of Myc epitopes in the C-terminal zone. Constructs containing labeling in their N-terminal region do not differentiate a fragmented protein from a complete one. With the construct of the present invention, only proteins that have been correctly processed and are complete could be purified. A schematic of the PW series plasmid constructs is shown in Figure 2.

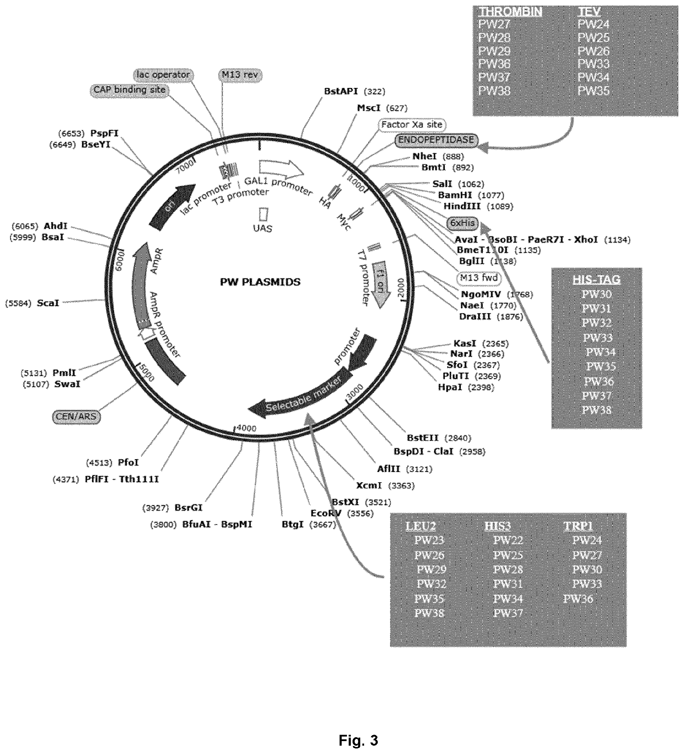

With all this, a total of 17 plasmids have been generated for yeast surface protein exposure that allow co-expression of two proteins simultaneously, recovery by protease treatment and subsequent purification by affinity chromatography. All constructs have been tested by sequencing and transformed into yeast strains of the invention. A representative map of the plasmids generated in the present invention can be seen in Figure 3. Table 6 shows a summary of the differential characteristics of the plasmids.

**Table 6. Set of plasmids obtained by genetic engineering with detailed information on their selection marker, added protease recognition site, and tag for purification.**

| NAME | TRP | HIS | LEU | TEV | THR | HIS TAG |
|---|---|---|---|---|---|---|
| PW22 | | x | | | | |
| PW23 | | | x | | | |
| PW24 | x | | | X | | |
| PW25 | | x | | X | | |
| PW26 | | | x | X | | |
| PW27 | x | | | | X | |
| PW28 | | x | | | X | |
| PW29 | | | x | | X | |
| PW30 | x | | | | | X |
| PW31 | | x | | | | X |
| PW32 | | | x | | | X |
| PW33 | x | | | X | | X |
| PW34 | | x | | X | | X |
| PW35 | | | x | X | | X |
| PW36 | x | | | | X | X |
| PW37 | | x | | | X | X |
| PW38 | | | x | | X | X |

### Obtaining yeast strains with enhanced surface display

The remarkable feature of these strains is that the natural promoter has been replaced by a Galactose-regulated promoter that has the sequence SEQ ID NO: 1. There is a strain similar to yK110 generated in the laboratory of Dane Wittrup, EBY100 (with genotype MATa AGA1::GAL1-AGA1::URA3 ura3-52 trp1 leu2-delta200 his3-delta200 pep4::HIS3 prbd1.6R can1 GAL), but in this case a Galactose-regulated AGA1 construct was inserted into the gene region corresponding to the URA3 gene. The difference is important, since without induction the surface is already covered with the natural AGA1-AGA2 complex, which reduces the possibility of protein exposure after induction. This was tested by flow cytometry with all three strains showing an increase in exposure of about 300% in yK401, and between 150- 250% in yK110 and yK501. The surface protein exposure efficiency is a limiting factor in the application of the technique that we have optimized significantly.

The cytometer results obtained in independent experiments are shown in Figure 4. The strain already described in Wittrup's work achieves a percentage of cells with YSD of 14.3% at 24 hours of induction, while the YK110 strain with a representative plasmid from the PW36 kit reaches 37.88%. In an independent experiment, the percentages of YSD cells of strains YK401 and YK501 with representative plasmids from the generated collection were evaluated, obtaining the following values; EBY100+pCTcon2 7.65%, YK401+PW37 24.17%, YK401+PW38 18.76%, YK501+PW37 20.88%. Considering the obtained values, the % increase of cells successfully expressing yeast surface proteins was calculated (Fig. 4).

### III - CONCLUSION

The present invention provides a set of plasmids and strains that improves and makes yeast surface exposure experiments more flexible. System optimization is essential for process reproducibility, as the ultimate goal is to scale up and robotize experiments. The reagents needed to perform surface exposure, extraction, and purification are very common in laboratories, can be purchased from various laboratory supply companies, and are inexpensive and accessible. The system developed here is simple and robust while combining its elements allows simple adaptation to the experimental objectives.

## Claims

1. A recombinant yeast **characterized in that** its genome comprises a metabolite-inducible promoter in place of the native promoter of the AGA1 gene.

2. Yeast according to claim 1, wherein the metabolite-inducible promoter is the galactose-inducible promoter.

3. Yeast according to claim 1 or 2, wherein the yeast belongs to the species *Saccharomyces cerevisiae.*

4. Yeast according to any one of claims 2 or 3, wherein the nucleotide sequence of the galactose-inducible promoter comprises the sequence SEQ ID NO: 1.

5. Yeast according to any one of claims 1 to 4, **characterized in that** it further comprises one or more plasmids, wherein each plasmid comprising:
(i) a metabolite-inducible promoter operably linked to the nucleotide sequence coding for a fusion protein, wherein said fusion protein comprises in the N-terminal - C-terminal direction: (a) the amino-terminal region of the Aga2 protein, (b) a first tag for immunological detection, (c) a protein of interest, (d) a second tag for immunological detection, and (e) a purification tag of the protein of interest;
(ii) a broad-range promoter functionally linked to a selection marker; and
(iii) a CEN/ARS centromeric origin of replication.

6. Yeast according to claim 5, wherein the first tag for immunological detection is an HA epitope.

7. Yeast according to claims 5 or 6, wherein the plasmid further comprises a protease recognition site preferably recognized by TEV protease or THR protease.

8. Yeast according to any one of claims 5 to 7, wherein the protein of interest is selected from the list consisting of an antibody or an antibody fragment, an enzyme with degradative activity, a protein adsorbing toxins or food contaminations, an enzyme with electron-accepting or electron-donating capacity.

9. Yeast according to any one of claims 5 to 8, wherein the second tag for immunological detection is a c-Myc epitope.

10. Yeast according to any one of claims 5 to 9, wherein the selection marker is an auxotrophic marker.

11. Yeast according to claim 10, wherein the auxotrophic marker is TRP, HIS or LEU.

12. Yeast according to any one of claims 5 to 11, wherein the purification tag of the protein of interest is a histidine tail.

13. Yeast according to any one of claims 5 to 12, wherein the plasmid comprises the nucleotide sequence SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

14. Yeast according to any one of claims 5 to 12, **characterized in that** it comprises two plasmids selected from the list consisting of SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) and SEQ ID NO: 19 (pCTcon2).

15. Yeast according to claim 14, wherein one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

16. Yeast according to claim 14, wherein one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

17. A composition comprising a yeast according to any one of claims 1 to 16.

18. A yeast library comprising a yeast according to any one of claims 1 to 16.

19. Use of a yeast according to any one of claims 1 to 16 in the yeast surface display technique (Yeast Surface Display).

20. Method for obtaining a yeast according to any one of claims 1 to 16, comprising a step (a) comprising genetic replacement by homologous recombination of the natural promoter of the AGA1 gene in yeast with a metabolite-induced promoter.

21. Method according to claim 20, wherein the yeast belongs to the species *S*. *cerevisiae.*

22. Method according to claim 20 or 21, wherein the metabolite-inducible promoter is the galactose-inducible promoter.

23. Method according to claim 22, wherein the nucleotide sequence of the galactose-inducible promoter comprises the sequence SEQ ID NO: 1.

24. Method according to any one of claims 20 to 23, further comprising a step (b) comprising transforming the yeast obtained in step (a) with one or more plasmids, wherein each plasmid comprises,
(i) a metabolite-inducible promoter operatively linked to the nucleotide sequence coding for a fusion protein, wherein said fusion protein comprises in N-terminal - C-terminal direction: (a) the amino-terminal region of the Aga2 protein, (b) a first tag immunological detection, (c) a protein of interest, (d) a second tag for immunological detection, and (e) a purification tag of the protein of interest;
(ii) a broad-spectrum promoter operationally linked to a selection marker; and (iii) a CEN/ARS centromeric origin of replication.

25. Method according to claim 24, wherein the first tag for immunological detection is an HA epitope.

26. Method according to claims 24 or 25, wherein the plasmid further comprises a protease recognition site preferably recognized by TEV protease or THR protease.

27. Method according to any one of claims 24 to 26, wherein the protein of interest is selected from the list consisting of an antibody or antibody fragment, an enzyme with degradative activity, a toxin- or food contamination-absorbing protein, an enzyme with electron-accepting or electron-donating capacity.

28. Method according to any one of claims 24 to 27, wherein the second immunological detection tag is a c-Myc epitope.

29. Method according to any one of claims 24 to 28, wherein the marker is an auxotrophic marker.

30. Method according to claim 29, wherein the auxotrophic marker is TRP, HIS or LEU.

31. Method according to any one of claims 24 to 30, wherein the purification tag of the protein of interest is a histidine tail.

32. Method according to any one of claims 24 to 31, further comprising a step (b) comprising transforming the yeast obtained in step (a) with one or more plasmids, wherein the plasmid(s) is/are selected from the list consisting of SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

33. Method according to claim 32, wherein one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

34. Method according to claim 32, wherein one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

35. A plasmid comprising: (i) a metabolite-inducible promoter operatively linked to the nucleotide sequence coding for a fusion protein, wherein said fusion protein comprises in N-terminal - C-terminal direction: (a) the amino-terminal region of the Aga2 protein, (b) a first tag for immunological detection, (c) a protein of interest, (d) a second tag for immunological detection, and (e) a purification tag of the protein of interest;
(ii) a broad-range promoter operationally linked to a selection marker; and (iii) a CEN/ARS centromeric origin of replication.

36. Plasmid according to claim 35, wherein the first tag for immunological detection is an HA epitope.

37. Plasmid according to claims 35 or 36, further comprising a protease recognition site preferably recognized by TEV protease or THR protease.

38. Plasmid according to any one of claims 35 to 37, wherein the protein of interest is selected from the list consisting of an antibody or antibody fragment, an enzyme with degradative activity, a toxin or food contamination absorbing protein, an enzyme with electron accepting or electron-donating capacity.

39. Plasmid according to any one of claims 35 to 38, wherein the second tag immunological detection is a c-Myc epitope.

40. Plasmid according to any one of claims 35 to 39, wherein the selection marker is an auxotrophic marker.

41. Plasmid according to claim 40, wherein the auxotrophic marker is TRP, HIS or LEU.

42. Plasmid according to any one of claims 35 to 41, wherein the purification tag of the protein of interest is a Histidine tail.

43. Plasmid according to any one of claims 35 to 42, **characterized in that** it comprises the sequence SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

44. A plasmid pair, wherein the plasmids are selected from the list consisting of SEQ ID NO: 2 (PW22), SEQ ID NO: 3 (PW23), SEQ ID NO: 4 (PW24), SEQ ID NO: 5 (PW25), SEQ ID NO: 6 (PW26), SEQ ID NO: 7 (PW27), SEQ ID NO: 8 (PW28), SEQ ID NO: 9 (PW29), SEQ ID NO: 10 (PW30), SEQ ID NO: 11 (PW31), SEQ ID NO: 12 (PW32), SEQ ID NO: 13 (PW33), SEQ ID NO: 14 (PW34), SEQ ID NO: 15 (PW35), SEQ ID BO: 16 (PW36), SEQ ID NO: 17 (PW37), SEQ ID NO: 18 (PW38) or SEQ ID NO: 19 (pCTcon2).

45. A plasmid pair according to claim 44, wherein one of the plasmids is selected from the list consisting of SEQ ID NO: 19 (pCTcon2), SEQ ID NO: 4 (PW24), SEQ ID NO: 7 (PW27), SEQ ID NO: 10 (PW30), SEQ ID NO: 13 (PW33) and SEQ ID NO: 16 (PW36); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

46. A plasmid pair according to claim 44, wherein one of the plasmids is selected from the list consisting of SEQ ID NO: 2 (PW22), SEQ ID NO: 5 (PW25), SEQ ID NO: 8 (PW28), SEQ ID NO: 11 (PW31), SEQ ID NO: 14 (PW34) and SEQ ID NO: 17 (PW37); and the other plasmid is selected from the list consisting of SEQ ID NO: 3 (PW23), SEQ ID NO: 6 (PW26), SEQ ID NO: 9 (PW29), SEQ ID NO: 12 (PW32), SEQ ID NO: 15 (PW35) and SEQ ID NO: 18 (PW38).

47. A host cell comprising a plasmid according to any one of claims 35 to 43, or a plasmid pair according to any one of claims 44 to 46.

48. Host cell according to claim 47, wherein the host cell is a yeast.

49. Host cell according to claim 48, wherein the yeast is *S*. *cerevisiae.*

50. Use of a plasmid according to any one of claims 35 to 43, or a plasmid pair according to any one of claims 44 to 46, for transforming a yeast, preferably a yeast according to any one of claims 1 to 4.

51. A method for exposing a protein of interest on the surface of a yeast comprising:
(i) transforming a recombinant yeast according to any one of claims 1 to 4 with one or more plasmids according to any one of claims 35 to 43, or with a pair of plasmids according to any one of claims 44 to 46, and
(ii) grow the yeast transformed in step (i) under conditions suitable for the expression of the protein(s) of interest on the yeast surface.

52. Method according to claim 51, wherein the protein of interest is selected from the list consisting of an antibody or antibody fragment, an enzyme with degradative activity, a protein absorbing toxins or food contaminations, an enzyme with electron-accepting or electron-donating capacity.

53. Method according to claim 51 or 52, wherein the transformation of the yeast in step (i) is carried out by chemical transformation with LiAc and PEG.

54. A kit comprising a yeast according to any one of claims 1 to 16, a composition according to claim 17, a yeast library according to claims 18, a plasmid according to any one of claims 35 to 43, a pair of plasmids according to any one of claims 44 to 46, and/or a host cell according to any one of claims 47 to 49.

55. Use of a kit according to claim 54 in a method for exposing a protein of interest on the surface of a yeast, or in a method for transforming a yeast, or in the yeast surface display technique, or in a method according to any one of claims 20 to 34.

56. Use according to claim 55, wherein the yeast is a yeast according to any one of claims 1 to 16.
